# EUROPÄISCHE PATENTSCHRIFT

(11) **EP 0 340 560 B1**
(45) Veröffentlichungstag und Bekanntmachung des Hinweises auf die Patenterteilung: **29.09.1993**
(21) Anmeldenummer: 89107204.3
(22) Anmeldetag: 21.04.1989
(51) Int. Cl.: C07C 205/15, C07C 201/12

(54) **Aldolkondensation von Nitroparaffinen**
Aldolcondensation of nitroparaffines
Condensation aldolique de nitroparaffines

(30) Priorität: 30.04.1988 DE 3814772
(43) Veröffentlichungstag der Anmeldung: 08.11.1989
(73) Patentinhaber: Henkel Kommanditgesellschaft auf Aktien, 40191 Düsseldorf (DE)
(72) Erfinder: Wüst, Willi, Dr., D-4030 Ratingen (DE); Eskuchen, Rainer, Dr., D-4000 Düsseldorf (DE); Esser, Herbert, D-5210 Troisdorf (DE)

(56) Entgegenhaltungen:
- DE-A- 1 910 458
- US-A- 2 301 259

## Beschreibung

Die Erfindung betrifft ein großtechnisch durchführbares Verfahren zur Aldolkondensation von Nitroparaffinen mit Aldehyden, insbesondere Formaldehyd.

Die Basen-katalysierte Reaktion von Nitroparaffinen mit Aldehyden, insbesondere Formaldehyd, ist bekannt. So können beispielsweise bei Nitromethan alle aciden Wasserstoffatome durch Formaldehyd in Gegenwart von Basen durch Hydroxymethyl-Gruppen substituiert werden. Die Reaktion ist stark exotherm; darüberhinaus können bei Überschreiten gewisser Temperaturgrenzen Nebenreaktionen auftreten, da sowohl die Aldehyde als auch die Nitroparaffine in Gegenwart von Basen bei erhöhten Temperaturen mit sich selbst reagieren. Es ist daher üblich, in organischem Lösungsmittel oder in hoher Verdünnung zu arbeiten.

Aus der japanischen Patentanmeldung 74/70,911, zitiert bei Chemical Abstracts 81, 119937y, (1974), wird die Umsetzung von wässriger Formaldehydlösung mit wässriger Natronlauge bei Temperaturen von 5 bis 10 °C in g-Mengen beschrieben.

Vor dem Hintergrund dieses Standes der Technik bestand ein Bedarf nach einem großtechnischen, kontinuierlichen oder diskontinuierlichen Verfahren zur Umsetzung von Nitroparaffinen mit Aldehyden. Eine weitere, sehr spezielle Aufgabe der Erfindung besteht darin, ein Verfahren zur Umsetzung von Nitromethan mit Formaldehyd in Gegenwart von Kalilauge und dessen großtechnische kontinuierliche oder diskontinuierliche Durchführung zu beschreiben.

Gegenstand der Erfindung ist somit ein Verfahren zur Umsetzung von Nitroparaffinen mit Formaldehyd in Gegenwart von Basen unter Aldolkondensation, wobei man unter Vermischung Nitroparaffin, wässrige Aldehydlösung und Alkalilauge in Abwesenheit organischer Lösungsmittel in einen Rohrreaktor einspeist und nach Passieren eines Wärmetauschers in ein Nachreaktionsgefäß leitet, wobei man über die gesamte Reaktionsstrecke die Temperatur in einer Bandbreite zwischen -5 und +20 °C einregelt.

Zur Durchführung des Verfahrens dosiert man zunächst die einzelnen Reaktionskomponenten in ein Reaktionsrohr. Nach einer bevorzugten Ausführungsform wird dazu zunächst der Aldehyd mit der Alkalilauge vermischt, wobei darauf zu achten ist, daß die Temperatur der Mischung nicht über 10 °C steigt, da ansonsten Disproportionierung (Cannizarro-Reaktion) auftreten kann. Zu dieser Mischung wird dann das Nitroparaffin dosiert und Durchmischung des Reaktionsgemisches bewirkt.

Nach einer anderen Ausführungsform der Erfindung können jedoch auch die Einzelkomponenten getrennt in das Reaktionsrohr dosiert werden und dort gleichzeitig vermischt werden. Dabei kann die Vermischung nach einer Ausführungsform der Erfindung im Schergefälle einer Pumpe durchgeführt werden, die über 2 oder 3 Ansaugstutzen die einzelnen Ausgangsstoffe ansaugt. Andererseits kann die Durchmischung auch in einem Reaktionsrohr mit eingebauten Schikanen, also in einem Statikmischer, durchgeführt werden. Schließlich kann die Durchmischung auch in einem Rührkessel bewerkstelligt werden, wobei jedoch, wie auch bei den anderen Mischvorrichtungen, die Wärmeabfuhr gewährleistet sein muß.

Durch den Rohrreaktor wird sodann die Reaktionsmischung zur Abführung der Reaktionsenthalpie in einen Wärmetauscher geführt. Nach einer bevorzugten Ausführungsform der Erfindung wird hierbei ein Plattenwärmetauscher eingesetzt, jedoch können auch Wärmetauscher anderer Bauart, so zum Beispeil Rohrbündelwärmetauscher, verwendet werden.

Bei der Durchführung des Verfahrens hat es sich als zweckmäßig erwiesen, die Temperatur der Reaktionsmischung an mehreren Stellen zu überwachen, um zu gewährleisten, daß über die Fließgeschwindigkeit der Gesamtmischung die Zone des höchsten Reaktionsumsatzes in dem Teil der Apparatur zu liegen kommt, in dem die Wärmeabfuhr am besten gewährleistet ist.

Aus dem Wärmetauscher wird die Reaktionsmischung in die Nachreaktionszone geführt. Die Nachreaktion ist nur noch mäßig exotherm. Als Nachreaktionszone kann eine Rohrstrecke des Rohrreaktors angesehen werden. Es ist jedoch auch möglich, die Nachreaktion in einem Rührkessel oder einer Rührkesselkaskade durchzuführen, oder auch im Ausgangsmischkessel.

Bei der Durchführung der Reaktion hat es sich als zweckmäßig erwiesen, den Wassergehalt des Gesamtsystems auf 45 bis 60 Gew.-%, insbesondere auf 48 bis 52 Gew.-%, einzustellen. Durch diese Wassermenge werden sowohl Raum-Zeitausbeuten als auch die Beherrschbarkeit der Reaktion unter technischen Bedingungen gefördert.

Zur Durchführung des erfindungsgemäßen Verfahrens können zahlreiche Nitroparaffine eingesetzt werden. Vorzugsweise werden Nitroparaffine mit 1 bis 8 C-Atomen eingesetzt, insbesondere niedere Nitroparaffine, so beispielsweise Nitromethan oder dessen höhere Homologe mit bis zu 4 C-Atomen.

Die dem erfindungsgemäßen Verfahren zugrundeliegende Reaktion ist Basen-katalysiert. Als Basen werden Alkalilaugen verwendet. Besonders geeignet ist KOH. Es hat sich gezeigt, daß die Kaliumsalze von beispielsweise 2-Nitropropandiol unter Reaktionsbedingen so gut im Reaktionsgemisch löslich sind, daß keine Auskristallisationseffekte zu beobachten sind, die den Wärmedurchgang und damit die Temperaturkontrolle erschweren würden.

Als Aldehyde werden im erfindungsgemäßen Verfahren vorzugsweise kurzkettige Aldehyde, insbesondere mit bis zu 3 C-Atomen, eingesetzt. Besonders bevorzugte Aldehyde sind Formaldehyd, Acetaldehyd oder auch Chloral.

Beim Einsatz von Nitromethan als Nitroparaffin und von Formaldehyd als Aldehyd läßt sich das Verfahren in unterschiedlicher Weise gestalten. So erhält man bei Zusatz katalytischer Basenmengen und einem ausreichenden Formaldehyd-Angebot als Reaktionsprodukt 2-(Hydroxymethyl)-2-nitro-propandiol. Diese Verbindung kann als Mikrobizid eingesetzt werden oder auch zu dem entsprechenden Amin reduziert werden, das vielfältige Verwendung, beispielsweise als Puffer oder auch in der Lackindustrie hat.

Bei Verwendung von Formaldehyd und Nitromethan kann andererseits in Gegenwart stöchiometrischer Basenmengen das Alkalimetallsalz der 2-Nitro-1,3-propandiols hergestellt werden. Diese Verbindung kann nach Neutralisation zu bekannten Mikrobiziden, zum Beispiel dem 2-Brom-2-nitro-1,3-propandiol weiterverarbeitet werden oder auch zum Amin reduziert werden.

Beim erfindungsgemäßen Verfahren ist eine strikte Kontrolle der Reaktionstemperatur angezeigt. So wird das Verfahren in einem Temperaturbereich von -5 bis +20 °C durchgeführt, vorzugsweise in einem Temperaturbereich von 0 bis 15 °C.

Die verfahrensgemäß erhaltenen alkalischen Lösungen werden, soweit sie nicht als solche für nachfolgende Reaktionsschritte eingesetzt werden, neutralisiert, um die Rückreaktion zu vermeiden. Die so erhaltenen Nitroalkohole, die im Neutralen bis Sauren stabil sind, können dann erforderlichenfalls in üblicher Weise abgetrennt werden.

Nach einer bevorzugten Ausgestaltungsform des erfindungsgemäßen Verfahrens wird in einem Reaktionskessel eine Mischung aus wässriger Aldehydlösung und der Alkalilauge bereitgestellt und mittels einer Pumpe über den Wärmetauscher, beispielsweise einen Plattenwärmetauscher, im Kreislauf in den Ausgangsreaktionskessel geführt. Man gibt dann vor der Pumpe die Nitroparaffin-Lösung in einer solchen Geschwindigkeit zu, daß die Reaktionstemperatur von maximal 20 °C, insbesondere maximal 15 °C, an keiner Stelle überschritten wird.

### Beispiele

### Beispiel 1

### Herstellung von 2-Nitro-propandiol-1,3-kaliumsalz

Gearbeitet wurde in einer Technikumsanlage, bestehend aus einem 0,8 m³ Rührwerksbehälter, einer Kreiselpumpe mit einem Durchsatz von 25 m³·h⁻¹; H = 40 m und einem 12 m² Plattenwärmetauscher.

In dem Rührwerksbehälter werden 229,2 kg 37 Gew.-%ige wässrige Formaldehydlösung vorgelegt und durch Umpumpen über den Wärmetauscher zurück in den Rührwerksbehälter auf eine Temperatur von 0 bis 10 °C gekühlt. Innerhalb von 30 min wurden dann 201,4 kg einer 45 Gew.-%igen wässrigen Kalilauge zudosiert, wobei die Vermischung im Schergefälle der Kreiselpumpe eintrat. Die Temperatur der Mischung wurde im Bereich von 0 bis 10 °C gehalten.

Zu dieser Mischung wurden innerhalb von 2 Stunden 82,1 kg Nitromethan zudosiert. Dabei wurde die Dosiergeschwindigkeit so eingestellt, daß die Temperatur der Reaktionsmischung an keiner Stelle 15 °C überstieg. Die Temperatur war über den Dosierstrom des Nitromethans regelbar. Die Nachreaktionszeit betrug 15 min, während denen das Reaktionsgemisch im Kreis gepumpt wurde. Am Ende der Reaktionszeit war der Restgehalt an Nitromethan auf unter 1 Gew.-% gefallen. Es konnte gezeigt werden, daß das gebildete Kaliumsalz des 2-Nitro-1,3-propandiols bei Temperaturen bis hinab zu -15 °C in Lösung blieb. Durch Neutralisation der Salzlösung ließ sich 2-Nitro-1,3-propandiol herstellen.

### Beispiel 2

Beispiel 1 wurde wiederholt, jedoch wurde anstelle von Kalilauge die äquivalente Menge Natronlauge eingesetzt. Als Reaktionsprodukt wurde eine Suspension des Natriumsalzes im Reaktionsmedium erhalten. An einigen Stellen kam es zu Belagbildungen im Wärmetauscher, so daß dieser nach jeder Charge gereinigt werden mußte.

## Patentansprüche

1. Verfahren zur Umsetzung von Nitroparaffinen mit Formaldehyd in Gegenwart von Basen unter Aldolkondensation, dadurch gekennzeichnet, daß man unter Vermischung Nitroparaffin, wässrige Aldehydlösung und Alkalilauge in Abwesenheit organischer Lösungsmittel in einen Rohrreaktor einspeist und nach Passieren eines Wärmetauschers in ein Nachreaktionsgefäß leitet, wobei man über die gesamte Reaktionsstrecke die Temperatur in einer Bandbreite zwischen -5 und +20 °C einregelt.

2. Verfahren nach Anspruch 1, dadurch gekennzeichnet, daß man Formaldehydlösung und Alkalilauge unter Kühlung auf eine Temperatur von 0 bis 10 °C vermischt und zu dieser Mischung das Nitroparaffin zudosiert, wobei eine Reaktionstemperatur von 20 °C, insbesondere 15 °C, nicht überstiegen werden darf.

3. Verfahren nach einem der Ansprüche 1 oder 2, dadurch gekennzeichnet, daß man die Gesamtwassermenge im Reaktionsgemisch auf 45 bis 60 Gew.-%, insbesondere 48 bis 52 Gew.-%, einstellt.

4. Verfahren nach einem der Ansprüche 1 bis 3, dadurch gekennzeichnet, daß man als Alkalilauge Kaliumhydroxidlösung und als Aldehyd Formaldehyd einsetzt.

5. Verfahren nach einem der Ansprüche 1 bis 4, dadurch gekennzeichnet, daß man als Nitroparaffin Nitroparaffine mit 1 bis 8 C-Atomen, insbesondere Nitromethan einsetzt.

6. Verfahren nach einem der Ansprüche 1 bis 5, dadurch gekennzeichnet, daß man in Gegenwart katalytischer Mengen an Alkalilauge Nitromethan zu 2-Hydroxymethyl-2-nitro-1,3-propandiol umsetzt.

7. Verfahren nach einem der Ansprüche 1 bis 5, dadurch gekennzeichnet, daß man in Gegenwart molarer Mengen an Alkalilauge Nitromethan zum Kaliumsalz des 2-Nitro-1,3-propandiols umsetzt.

8. Verfahren nach einem der Ansprüche 1 bis 7, dadurch gekennzeichnet, daß man die Vermischung der Reaktionskomponenten im Schergefälle einer Kreiselpumpe oder bei Verwendung mehrerer Pumpen in einer statischen Mischstrecke durchführt.

9. Verfahren nach einem der Ansprüche 1 bis 8, dadurch gekennzeichnet, daß man als Wärmetauscher Plattenwärmetauscher einsetzt.

10. Verfahren nach einem der Ansprüche 1 bis 9, dadurch gekennzeichnet, daß man die Reaktionslösungen vorkühlt und die Reaktionstemperatur auf Werte zwischen 0 und 15 °C einstellt.

11. Verfahren nach einem der Ansprüche 1 bis 10, dadurch gekennzeichnet, daß man die Nachreaktion in einem Reaktionsrohr oder einem kühlbaren Reaktionskessel oder einer Kesselkaskade durchführt.

12. Verfahren nach einem der Ansprüche 1 bis 11, dadurch gekennzeichnet, daß man in einem Reaktionskessel eine Mischung von Aldehydlösung und Alkalilauge bereitstellt, diese über eine Wärmetauscher im Kreis pumpt und vor oder in der Pumpe das Nitroparaffin mit solcher Geschwindigkeit zudosiert, daß die gewünschte Reaktionstemperatur nicht überschritten wird.

## Claims

1. A process for reacting nitroparaffins with formaldehyde in the presence of bases by aldol condensation, characterized in that nitroparaffin, aqueous aldehyde solution and alkali metal hydroxide are fed with mixing into a tube reactor in the absence of organic solvents and, after passing through a heat exchanger, are introduced into an after-reaction vessel, the temperature being kept between -5 and +20°C throughout the reaction zone.

2. A process as claimed in claim 1, characterized in that formaldehyde solution and alkali metal hydroxide are mixed while cooling to a temperature of 0 to 10°C and the nitroparaffin is introduced into the resulting mixture, the reaction temperature not exceeding 20°C and, more particularly, 15°C.

3. A process as claimed in claim 1 or 2, characterized in that the total quantity of water in the reaction mixture is adjusted to 45 to 60% by weight and more particularly to 48 to 52% by weight.

4. A process as claimed in any of claims 1 to 3, characterized in that potassium hydroxide solution is used as the alkali metal hydroxide and formaldehyde is used as the aldehyde.

5. A process as claimed in any of claims 1 to 4, characterized in that a nitroparaffin containing 1 to 8 carbon atoms, more particularly nitromethane, is used as the nitroparaffin.

6. A process as claimed in any of claims 1 to 5, characterized in that nitromethane is reacted to form 2-hydroxymethyl-2-nitro-1,3-propanediol in the presence of catalytic quantities of alkali metal hydroxide.

7. A process as claimed in any of claims 1 to 5, characterized in that nitromethane is reacted to form the potassium salt of 2-nitro-1,3-propanediol in the presence of molar quantities of alkali metal hydroxide.

8. A process as claimed in any of claims 1 to 7, characterized in that the reaction components are mixed in the shear gradient of a rotary pump or, where several pumps are used, in a static mixing zone.

9. A process as claimed in any of claims 1 to 8, characterized in that a plate-type heat exchanger is used as the heat exchanger.

10. A process as claimed in any of claims 1 to 9, characterized in that the reaction solutions are precooled and the reaction temperature is adjusted to a value of 0 to 15°C.

11. A process as claimed in any of claims 1 to 10, characterized in that the after-reaction is carried out in a reaction tube or in a coolable reaction vessel or cascade of reaction vessels.

12. A process as claimed in any of claims 1 to 11, characterized in that a mixture of aldehyde solution and alkali metal hydroxide is prepared in a reaction vessel, pump-circulated through a heat exchanger and, before or in the pump, the nitroparaffin is added at such a rate that the desired reaction temperature is not exceeded.

## Revendications

1. Procédé de mise en réaction de nitroparaffines avec le formaldéhyde en présence de base dans les conditions de la condensation aldolique, caractérisé en ce que l'on alimente, tout en mélangeant la nitroparaffine, une solution aqueuse de formaldéhyde et la lessive alcaline en l'absence de solvant organique dans un réacteur tubulaire et qu'après traversée d'un échangeur de chaleur, on conduit dans un récipient de réaction finale, dans lequel on ajuste sur la totalité de l'étendue de la réaction la température dans une largeur de bande comprise entre -5° et + 20°C.

2. Procédé selon la revendication 1, caractérisé en ce que l'on mélange la solution de formaldéhyde et la lessive alcaline tout en refroidissant à une température de 0 à 10°C et que l'on ajoute par portions à ce mélange la nitroparaffine, une température de 20°C - en particulier de 15°C - ne devant pas être dépassée.

3. Procédé selon une des revendications 1 ou 2, caractérisé en ce que l'on ajuste la quantité totale d'eau dans le mélange réactionnel de 45 à 60 % en poids - en particulier de 48 à 52 % en poids -.

4. Procédé selon l'une des revendications 1 à 3, caractérisé en ce que comme lessive alcaline, on met en jeu une solution d'hydroxyde de potassium et comme aldéhyde, du formaldéhyde.

5. Procédé selon l'une des revendications 1 à 4, caractérisé en ce que l'on met en oeuvre comme nitroparaffine une nitroparaffine ayant de 1 à 8 atomes de carbone, en particulier le nitrométhane.

6. Procédé selon l'une des revendications 1 à 5, caractérisé en ce que l'on transforme en présence de quantités catalytiques de lessive alcaline, du nitrométhane en 2-hydroxyméthyl-2-nitro 1,3-propanediol.

7. Procédé selon l'une des revendications 1 à 5, caractérisé en ce que l'on fait réagir en présence de quantités molaires de lessive alcaline, le nitrométhane, en sel de potassium du 2-nitro 1,3-propanediol.

8. Procédé selon l'une des revendications 1 à 7, caractérisé en ce que l'on effectue le mélange des composants de la réaction dans le gradient de cisaillement d'une pompe centrifuge ou en utilisant plusieurs pompes dans un segment statique de mélange.

9. Procédé selon l'une des revendications 1 à 8, caractérisé en ce que l'on met en oeuvre comme échangeur de chaleur un échangeur de chaleur à plaques.

10. Procédé selon l'une des revendications 1 à 9, caractérisé en ce que l'on refroidit les solutions réactionnelles et que l'on ajuste la température de réaction à des valeurs comprises entre 0 et 15°C.

11. Procédé selon l'une des revendications 1 à 10, caractérisé en ce que l'on effectue la réaction finale dans un tube de réaction ou dans un récipient de réaction que l'on peut refroidir ou dans une cascade de récipients.

12. Procédé selon l'une des revendications 1 à 11, caractérisé en ce que l'on prépare dans un récipient pour réaction, un mélange de solution d'aldéhyde et de lessive alcaline, que l'on pompe ce mélange dans le cycle par l'intermédiaire d'un échangeur de chaleur et avant la pompe ou dans la pompe, que l'on ajoute par fractions la nitroparaffine avec une vitesse telle que la température de réaction désirée ne soit pas dépassée.
